# EUROPEAN PATENT SPECIFICATION

(11) **EP 4 316 401 B1**
(45) Date of publication and mention of the grant of the patent: **10.06.2026**
(21) Application number: 21948382.3
(22) Date of filing: 30.06.2021
(51) Int. Cl.: A61B 34/30, A61B 34/00

(54) **SURGICAL INSTRUMENT**
CHIRURGISCHES INSTRUMENT
INSTRUMENT CHIRURGICAL

(43) Date of publication of application: 07.02.2024
(73) Proprietor: RIVERFIELD Inc., 1070052 Tokyo (JP)
(72) Inventor: GOTANI, Yoshiki, Tokyo 1070052 (JP)
(74) Representative: Adares PartGmbB
(86) International application number: PCT/JP2021/024855
(87) International publication number: WO 2023/276077

(56) References cited:
- WO-A1-2014/151952
- CN-A- 107 049 477
- CN-A- 107 049 477
- CN-A- 109 009 414
- JP-B1- 6 850 517
- JP-B1- 6 850 517
- US-A1- 2018 153 627
- US-A1- 2021 038 215

## Description

### TECHNICAL FIELD

The present invention relates to the technical field of surgical instruments attached to surgical robot arms.

### BACKGROUND ART

In recent years, surgical operations using surgical robots are becoming popular in surgical systems. Some of such surgical robots are provided with robot arms, and various surgical instruments may be detachably attached to the robot arms.

A surgical instrument includes, for example, a base body attached to a robot arm and an end effector provided at a tip end portion, with scissors, forceps, a hook, a high-frequency knife, clamps, stapler, or the like being used as the end effector (see, for example, Patent Literature 1).

Surgery using a surgical robot as described above is generally performed by remotely operating a surgical robot installed in an operating room by an operator (doctor) using a master-slave system. In such surgical operations, for example, a surgical instrument is inserted into a patient's body from a port formed in a body cavity, and the surgical instrument is operated to treat an affected area, observe the affected area, or the like.

The surgical instrument described in Patent Literature 1 uses scissors as an end effector for performing excision or the like of an affected area, and the end effector has a first jaw and a second jaw. The first jaw is provided with a first pulley and the second jaw is provided with a second pulley, one end portion of a first wire is connected to the first pulley, and one end portion of a second wire is connected to the second pulley.

Therefore, in the surgical instrument described in Patent Literature 1, the first jaw is operated when the first pulley is rotated by pulling the first wire, the second jaw is operated when the second pulley is rotated by pulling the second wire, and the tip end portion of the first jaw and the tip end portion of the second jaw are moved in a direction toward and away from each other.

### CITATION LIST

### PATENT LITERATURE

Patent Literature 2: US 2018/153627 A1
Patent Literature 3: CN 107 049 477 A
Patent Literature 4: JP 6 850517 B1
Patent Literature 5: WO 2014/151952 A1

### SUMMARY OF INVENTION

### TECHNICAL PROBLEM

In a surgical instrument as described in Patent Literature 1, the first jaw and the second jaw are operated during surgery, and thus ensuring a stable and highly accurate state of operation of the end effector is required from the aspect of performing highly accurate surgery.

In a case where scissors are used as the end effector, in order to ensure good sharpness against shear resistance during the closing operation of the first jaw and the second jaw, it is desirable that a force be applied to the first jaw and the second jaw in a direction in which the first and the second jaw come in contact with each other; however, when excessive force is applied to the first jaw and the second jaw in a direction in which the first jaw and the second jaw are in contact with each other, smooth opening operation of the first jaw and the second jaw may be hindered.

Patent Literature 2 describes a surgical tool with an end effector having jaws which can perform opening and closing movements. The surgical tool further has elongate flexible members such as wires and a first plurality of pulleys at a wrist and a second plurality of pulleys at another wrist and can include a tool housing for being coupled to a surgical robot. The surgical tool has a first pivot axis about which the jaws pivot relative to each other to move the end effector between open and closed positions by actuation of the elongate flexible members. The first jaw has a first pair of the grooves formed therein that seat the flexible members, and the second jaw has a second pair of the grooves formed therein that seat the flexible members.

Patent Literature 3 describes positioning guided surgical electrocoagulation scissors, comprising a front end scissor mechanism comprising a pair of scissor bodies and a middle support and a control mechanism controlling the opening, closing and same direction swinging of the pair of scissor bodies through first and second steel wire ropes, and controlling the swinging of the middle support through a third steel wire rope. The control mechanism comprises a pitch active guide wheel group, a left active guide wheel group, and a right active guide wheel group. When passive pulleys rotate, they drive the corresponding steel wire ropes fixed thereon clockwise and counterclockwise to tighten and relax, so that the left active guide wheel group and the right active guide wheel group drive the left scissors body and the right scissors body to complete the closing and opening movement of the front pair of scissors bodies, and the pitch active guide wheel group drives the front pair of scissors bodies to swing back and forth.

Patent Literature 4 describes a forceps device comprising gripping sections, a support holding the gripping sections, a rotation shaft rotatably supporting the support, a base component holding the rotation shaft, and a plurality of guide pulleys disposed coaxially with the rotation shaft.

Patent Literature 5 describes a similar tool including an end effector with a pair of jaws, pulleys, and cables.

Therefore, an object of the surgical instrument of the present invention is to ensure a stable and highly accurate operating state of the end effector.

### SOLUTION TO PROBLEM

The invention is defined by the appended claims. First, a surgical instrument according to the present invention is a surgical instrument attached to a surgical robot arm, the surgical instrument including: a base body detachable from the robot arm; a support body connected to the base body; and an end effector including a first jaw and a second jaw that are each rotatably supported by the support body with a rotating shaft as a fulcrum; wherein the first jaw includes a first opening pulley to which a first opening wire is connected, a first closing pulley to which a first closing wire is connected, and a first blade part operated in an opening and closing direction; the second jaw includes a second opening pulley to which a second opening wire is connected, a second closing pulley to which a second closing wire is connected, and a second blade part operated in the opening and closing direction; the first opening pulley and the first closing pulley are each supported by the rotating shaft in a state of being separated from each other in an axial direction of the rotating shaft; the second opening pulley and the second closing pulley are each supported by the rotating shaft in a state of being separated from each other in the axial direction of the rotating shaft; the first blade part and the second blade part are operated in opening directions separating from each other by pulling the first opening wire and the second opening wire, respectively, and are operated in closing directions approaching each other by pulling the first closing wire and the second closing wire, respectively; the first opening pulley is positioned closer to the first blade part side than the second blade part with respect to the first closing wire and the second closing wire, respectively; the first opening pulley is positioned closer to the first blade part side than the second blade part with respect to the first closing pulley in a direction orthogonal to the opening and closing direction; and the second opening pulley is positioned closer to the second blade part side than the first blade part with respect to the second closing pulley in a direction orthogonal to the opening and closing direction.

As a result, due to moments generated during an opening operation of the first jaw and the second jaw, forces are applied to the first blade part and the second blade part in directions orthogonal to the opening and closing direction and in directions separating from each other, and due to moments generated during a closing operation of the first jaw and the second jaw, forces are applied to the first blade part and the second blade part in directions orthogonal to the opening and closing direction in directions to contact with each other.

Second, in the surgical instrument according to the present invention described above, the second opening pulley or the second closing pulley is positioned between the first opening pulley and the first closing pulley; and the first opening pulley or the first closing pulley is positioned between the second opening pulley and the second closing pulley.

As a result, since the pulleys of the first jaw and the pulleys of the second jaw are alternately positioned, a space for arranging the four pulleys in the axial direction of a rotating shaft can be reduced, and it becomes possible to position the two pulleys of the first jaw and the two pulleys of the second jaw separately.

Third, in the surgical instrument according to the present invention described above, the first opening pulley, the second closing pulley, the first closing pulley, and the second opening pulley are positioned in order in the axial direction of the rotating shaft; and in a direction orthogonal to the opening and closing direction, the first opening pulley is positioned on the first blade part side, and the second opening pulley is positioned on the second blade part side.

As a result, since the first opening pulley and the second opening pulley are positioned on both sides of the second closing pulley and the first closing pulley, the configuration is such that it is possible to increase the moments, particularly in the opening operation.

Fourth, in the surgical instrument according to a non-inventive embodiment, the second closing pulley, the first opening pulley, the second opening pulley, and the first closing pulley are positioned in order in the axial direction of the rotating shaft; and in a direction in which the first blade part and the second blade part are arranged, the second closing pulley is positioned on the first blade part side, and the first closing pulley is positioned on the second blade part side.

As a result, since the second closing pulley and the first closing pulley are located on both sides of the first opening pulley and the second opening pulley, the configuration is such that it is possible to increase the moments, particularly in the closing operation.

Fifth, in the surgical instrument according to the present invention described above, the first jaw is provided with a first base portion, and the first opening pulley and the first closing pulley protrude in a same direction from both end portions of the first base portion; and the second jaw is provided with a second base portion, and the second opening pulley and the second closing pulley protrude in a same direction from both end portions of the second base portion.

As a result, since both the first jaw and the second jaw are formed in a bifurcated shape, it is possible to alternately position the pulleys of the first jaw and the pulleys of the second jaw with a simple structure.

Sixth, in the surgical instrument according to the present invention described above, preferably the first base portion, the first blade part, the first opening pulley, and the first closing pulley are integrally formed; and the second base portion, the second blade part, the second opening pulley, and the second closing pulley are integrally formed.

As a result, the first jaw and the second jaw are each formed by one member.

Seventh, in the surgical instrument according to the present invention described above, preferably the support body is rotatably connected to the base body with a fulcrum shaft as a fulcrum; and the axial direction of the rotating shaft and the axial direction of the fulcrum shaft are orthogonal to each other.

As a result, the end effector is allowed to move in two orthogonal directions.

### ADVANTAGEOUS EFFECTS OF INVENTION

With the present invention, moments that are generated in the opening operation of the first jaw and the second jaw apply forces in directions orthogonal to the opening and closing direction to the first blade part and the second blade part in directions separating from each other, and moments that are generated in the closing operation of the first jaw and the second jaw apply forces in directions orthogonal to the opening and closing direction to the first blade part and the second blade part in directions to contact with each other, and thus a stable and highly accurate operating state of the end effector can be ensured.

### BRIEF DESCRIPTION OF DRAWINGS

FIG. 1, together with FIGS. 2 to 13, illustrates an embodiment of a surgical instrument of the present invention, and is a schematic perspective view illustrating a surgical robot and the like in a state with the surgical instrument attached.
FIG. 2, together with FIGS. 3 to 7, illustrates the surgical instrument according to the first embodiment, and is a side view.
FIG. 3 is a plan view.
FIG. 4 is a perspective view illustrating a state in which a first blade part and a second blade part are opened.
FIG. 5 is a perspective view illustrating a state in which the first blade part and the second blade part are closed.
FIG. 6 is a conceptual diagram for explaining moments generated in an opening operation and forces generated in the blade parts.
FIG. 7 is a conceptual diagram for explaining moments generated in a closing operation and forces generated in the blade parts.
FIG. 8, together with FIGS. 9 to 13, illustrates a surgical instrument according to a second embodiment, and is a side view.
FIG. 9 is a plan view.
FIG. 10 is a perspective view illustrating a state in which the first blade part and the second blade part are opened.
FIG. 11 is a perspective view illustrating a state in which the first blade part and the second blade part are closed.
FIG. 12 is a conceptual diagram for explaining moments generated in an opening operation and forces generated in the blade parts.
FIG. 13 is a conceptual diagram for explaining moments generated in a closing operation and forces generated in the blade parts.

### DESCRIPTION OF EMBODIMENTS

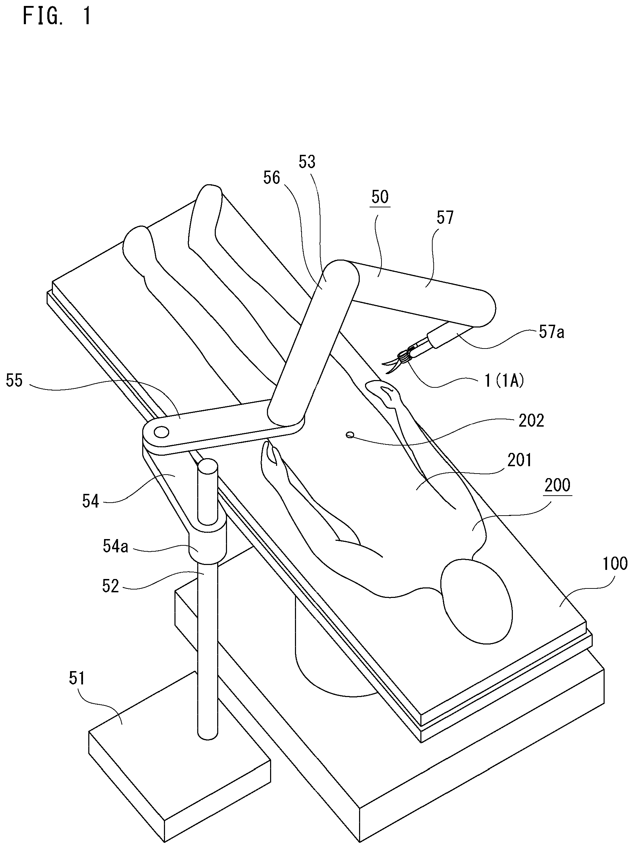

Embodiments of a surgical instrument of the present invention will be described below with reference to the accompanying drawings (see FIGS. 1 to 13).

The embodiments described below are examples in which the surgical instrument of the present invention is applied to a type that is used by being installed on the floor or the like of an operating room. However, the scope of application of the surgical instrument of the present invention is not limited to a type that is used by being installed on the floor or the like of an operating room, and the surgical instrument of the present invention may also be applied to a type that is used by being attached to a ceiling or a wall of an operating room.

Note that the surgical instrument described below, together with being formed in a shape having a longitudinal direction, and is provided in a state in which a plurality of pulleys are arranged side by side on the end effector, and hereinafter, the longitudinal direction is defined as the front-rear direction, and the direction in which the plurality of pulleys are arranged is defined as the vertical direction. However, the front, rear, up, down, left, and right directions below are for convenience of explanation, and the implementation of the present invention is not limited to these directions.

### <Schematic Configuration of Surgical Robot>

First, a schematic configuration of an example of a surgical robot 50 to which a surgical instrument 1 or a surgical instrument 1A is attached will be described (see FIG. 1). Note that, as will be described later, the surgical instrument 1 is the surgical instrument according to a first embodiment, and the surgical instrument 1A is the surgical instrument according to a second embodiment.

An operating table 100 is installed in an operating room, and a patient 200 is laid on the operating table 100, for example, in a supine position. A port 202 is formed in a body cavity 201 of the patient 200, for example, in the abdominal wall. A portion (tip end portion) of the surgical instrument 1 is inserted into the port 202 when a surgical operation is performed. The port 202 is a small hole into which the surgical instrument 1 is inserted.

A surgical robot 50 has a base 51 placed on the floor or the like of an operating room, a shaft-shaped pole 52 fixed to the base 51, and a main body 53 supported by the pole 52. The base 51 is installed on the floor or the like on a side of the operating table 100, and a lower end portion of the pole 52 is fixed to the base 51 while extending vertically.

The main body 53 has, for example, a first support arm 54, a second support arm 55, a first connecting arm 56 and a second connecting arm 57. The first support arm 54, the second support arm 55, the first connecting arm 56 and the second connecting arm 57 function as robot arms.

The first support arm 54 is formed in a shape extending in the horizontal direction, and one end portion in the longitudinal direction is provided as a supported cylindrical portion 54a. By inserting the pole 52 through the supported cylindrical portion 54a, the first support arm 54 is able to move in the vertical direction with respect to the pole 52 manually or electrically, and is able to rotate with respect to the pole 52 in a direction about the axis of rotation with the supported cylindrical portion 54a functioning as a fulcrum.

The first support arm 54 may be fixed to the pole 52 at a desired vertical position and a desired position about the axis of rotation.

The second support arm 55 is formed in a shape extending in the horizontal direction, and one end portion in the longitudinal direction is rotatably connected to an other end portion in the longitudinal direction of the first support arm 54. The second support arm 55 is rotatable with respect to the first support arm 54 manually or electrically.

By having one end portion in the longitudinal direction connected to the other end portion in the longitudinal direction of the second support arm 55, the first connecting arm 56 is able to rotate with respect to the second support arm 55 and is able to rotate about an axis of rotation extending in the longitudinal direction. The first connecting arm 56 is manually or electrically rotated with respect to the second support arm 55 or rotated about the axis of rotation.

By having one end portion in the longitudinal direction connected to the other end portion in the longitudinal direction of the first connecting arm 56, the second connecting arm 57 is able to rotate with respect to the first connecting arm 56 and is able to rotate about an axis of rotation extending in the longitudinal direction. The second connecting arm 57 is manually or electrically rotated with respect to the first connecting arm 56 or rotated about the axis of rotation. The second connecting arm 57 is provided with an other end portion in the longitudinal direction as a mounting portion 57a.

Note that in the above description, the surgical robot 50 having the configuration in which the first support arm 54, the second support arm 55, the first connecting arm 56, and the second connecting arm 57 are connected in order is shown as an example; however, the number of robot arms (support arms and connecting arms) provided in the surgical robot 50 is arbitrary, as long as the surgical robot 50 is provided with at least one robot arm.

### <Operation of Surgical Robot>

Next, an outline of the operation of the surgical robot 50 to which the surgical instrument 1 or a surgical instrument 1A is attached will be described.

The surgical robot 50 is used in surgical operations; for example, the surgical robot 50 installed in an operating room is remotely operated by an operator (doctor) using a master-slave system. A master-slave system is a method in which roles are shared between a master device that controls and operates multiple devices and a slave device that is operated under the control of the master device when multiple devices are operated in cooperation, and in a surgical operation, an operating device (control device) (not shown) operated by an operator is used as a master device, and the surgical robot 50 is used as a slave device.

In a surgical operation, when an operator inputs an operation signal to be executed by the surgical robot 50 to the operating device, the operation signal is transmitted from the operating device to the surgical robot 50 via a control portion (not shown). When an operation signal is input to the surgical robot 50, the first connecting arm 56, the second connecting arm 57, the surgical instrument 1, and the like are operated according to the input operation signal. Note that the surgical robot 50, for example, may be provided with a camera unit that functions as an endoscope, and may be configured such that the camera unit takes pictures in accordance with the input operation signal.

When using the surgical robot 50 in a surgical operation, first, the first connecting arm 56 is moved in a vertical direction with respect to the pole 52 according to a position of the patient 200 to set the vertical position of the main body 53, then, according to the position of the surgical procedure, the first connecting arm 56 is rotated with respect to the pole 52 and the second connecting arm 57 is rotated with respect to the first connecting arm 56 to set the position of the surgical instrument 1.

When a surgical operation is performed, a pivot point is set as a reference point for movement of the surgical instrument 1. The pivot point is a position that approximately coincides with the port 202 into which the surgical instrument 1 is inserted, and in a case where a trocar is used, approximately coincides with a position of the trocar. Accordingly, in a state in which the surgical instrument 1 is inserted into the body cavity 201 of the patient 200, a proper pivot operation is performed by controlling the position of the surgical instrument 1 such that a portion of the surgical instrument 1 always passes through the pivot point. By performing a proper pivot operation, the position of the surgical instrument 1 at the pivot point is not changed, and the occurrence of load on tissues near the body surface of the patient 200 is prevented, ensuring safety.

### <Configuration of Surgical Instrument of the First Embodiment>

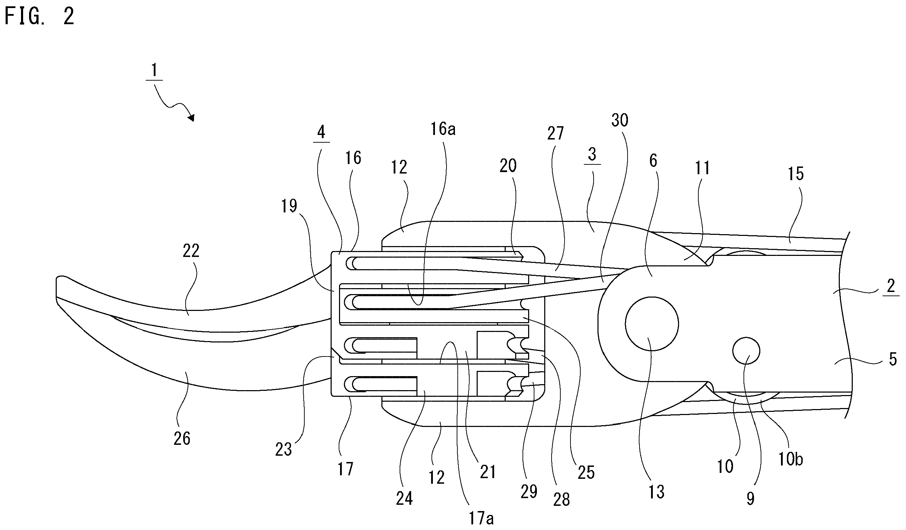

Next, the configuration of the surgical instrument 1 of the first embodiment will be described (see FIG. 2 to FIG. 5).

The surgical instrument 1 is detachable from the mounting portion 57a of the second connecting arm 57. The surgical instrument 1 has a base body 2, a support body 3 and an end effector 4.

The base body 2 has a substantially cylindrical mounted portion 5, the axial direction of which is oriented in the front-rear direction, and support arm portions 6, 6, each protrude forward from a front end portion of the mounted portion 5. The mounted portion 5 is a portion attached to the mounting portion 57a of the second connecting arm 57 in the surgical robot 50. The support arm portions 6, 6 protrude forward from positions on 180-degree opposite sides of the mounted portion 5.

Both end portions in the axial direction of a first support shaft 7 are connected to rear end portions of the support arm portions 6, 6. The axial direction of the first support shaft 7 is the left-right direction. First auxiliary pulleys 8, 8 are rotatably supported at positions near both ends in the axial direction of the first support shaft 7. The first auxiliary pulleys 8, 8 are positioned near inner surfaces of the support arm portions 6, 6, respectively.

Second support shafts 9, 9 are connected to central portions in the front-rear direction of the support arm portions 6, 6, respectively. The axial direction of each second support shaft 9 is the left-right direction. Second auxiliary pulleys 10, 10 are rotatably supported on the second support shafts 9, 9, respectively. Each second auxiliary pulley 10 is composed of two pulley portions 10a, 10b that are aligned in the axial direction and positioned near the inner surface of the support arm portion 6.

In the second auxiliary pulleys 10, 10, pulley portions 10a, 10a are positioned inside in the left-right direction, and pulley portions 10b, 10b are positioned outside of the pulley portions 10a, 10a in the left-right direction.

The support body 3 is composed of a wire support portion 11 and pulley support portions 12, 12, and the pulley support portions 12, 12 protrude forward from both left and right end portions of the wire support portion 11, respectively.

The wire support portion 11 is formed in a flat shape with the left-right direction being the thickness direction, and has a wire connecting groove 11a in a central portion in the thickness direction.

The pulley support portions 12, 12 protrude forward from both upper and lower end portions of the wire support portion 11, respectively.

Both end portions in the axial direction of fulcrum shafts 13, 13 are connected to the front end portions of the support arm portions 6, 6 and the wire support portion 11, respectively. The axial direction of each fulcrum shaft 13 is the left-right direction. Third auxiliary pulleys 14, 14 are rotatably supported by the fulcrum shafts 13, 13, respectively. Each third auxiliary pulley 14 is composed of two pulley portions 14a, 14b aligned in the axial direction.

In the third auxiliary pulleys 14, 14, the pulley portions 14a, 14a are positioned inside in the left-right direction, and the pulley portions 14b, 14b are positioned outside the pulley portions 14a, 14a in the left-right direction.

A support body wire 15 is inserted into the wire connecting groove 11a formed in the wire support portion 11 of the support body 3, and the support body wire 15 is wound around the wire support portion 11. When the support body wire 15 is pulled in one direction, the support body 3 is rotated about the fulcrum shaft 13 as a fulcrum in a direction in which the front end portion is moved upward with respect to the base body 2; and when the support body wire 15 is pulled in the other direction, the support body 3 is rotated about the fulcrum shaft 13 as a fulcrum in a direction in which the front end portion is moved downward with respect to the base body 2.

The end effector 4 is composed of a first jaw 16 and a second jaw 17. The first jaw 16 is, for example, a portion that constitutes a left (L side) scissor, and the second jaw 17 is, for example, a portion that constitutes a right (R side) scissor.

The end effector 4 is rotatably supported by the support body 3 with a rotating shaft 18 as a fulcrum. In the rotating shaft 18 the axial direction is the vertical direction, and both end portions in the axial direction are connected to the pulley support portions 12, 12 of the support body 3, respectively.

The first jaw 16 includes a first base portion 19 formed in a plate shape facing in a substantially front-rear direction, a first opening pulley 20 protruding rearward from an upper end portion of the first base portion 19, a first closing pulley 21 protruding rearward from a lower end portion of the first base portion 19, and a first blade part 22 protruding forward from a position near an upper end of the first base portion 19.

In the first jaw 16, the first base portion 19, the first opening pulley 20, the first closing pulley 21, and the first blade part 22 are integrally formed, for example.

As described above, in the first jaw 16, the first base portion 19, the first blade part 22, the first opening pulley 20, and the first closing pulley 21 are integrally formed, and thus the first jaw 16 is formed of one member, and the manufacturing cost of the surgical instrument 1 may be reduced.

In the first jaw 16, by passing the rotating shaft 18 through central portions of the first opening pulley 20 and the first closing pulley 21, the first jaw 16 is rotatable with respect to the support body 3 with the rotating shaft 18 as a fulcrum.

As described above, the axial direction of the rotating shaft 18 is vertically oriented, and the support body 3 is rotated with respect to the base body 2 about the fulcrum shaft 13 as a fulcrum, the axial direction of which is oriented in the left-right direction. Therefore, the direction of rotation of the first jaw 16 with respect to the support body 3 and the direction of rotation of the support body 3 with respect to the base body 2 are orthogonal to each other.

In the first jaw 16, the first opening pulley 20 and the first closing pulley 21 protrude rearward from both the upper and lower end portions of the first base portion 19, and thus the first jaw 16 has a space between the first opening pulley 20 and the first closing pulley 21, and this space is formed as a first space 16a.

The first blade part 22 is formed, for example, in a downward convex curved shape, and is provided as one blade part for performing excision or the like of an affected area of a patient. The first blade part 22 protrudes forward from a position closer to the first opening pulley 20 side in a direction in which the first opening pulley 20 and the first closing pulley 21 are aligned.

The second jaw 17 includes a second base portion 23 formed in a plate shape facing in a substantially front-rear direction, a second opening pulley 24 protruding rearward from a lower end portion of the second base portion 23, a second closing pulley 25 protruding rearward from an upper end portion of the second base portion 23, and a second blade part 26 protruding forward from a position near a lower end of the second base portion 23.

In the second jaw 17, the second base portion 23, the second opening pulley 24, the second closing pulley 25, and the second blade part 26 are integrally formed, for example.

As described above, in the second jaw 17, the second base portion 23, the second blade part 26, the second opening pulley 24, and the second closing pulley 25 are integrally formed, and thus the second jaw 17 may be formed of one member, and the manufacturing cost of the surgical instrument 1 may be reduced.

In the second jaw 17, by passing the rotating shaft 18 through central portions of the second opening pulley 24 and the second closing pulley 25, the second jaw 17 is rotatable with respect to the support body 3 with the rotating shaft 18 as a fulcrum.

As described above, the axial direction of the rotating shaft 18 is vertically oriented, and the support body 3 is rotated with respect to the base body 2 about the fulcrum shaft 13 as a fulcrum, the axial direction of which is oriented in the left-right direction. Therefore, the direction of rotation of the second jaw 17 with respect to the support body 3 and the direction of rotation of the support body 3 with respect to the base body 2 are orthogonal to each other.

In the second jaw 17, the second closing pulley 25 and the second opening pulley 24 protrude rearward from both the upper and lower end portions of the second base portion 23, and thus the second jaw 17 has a space between the second opening pulley 24 and the second closing pulley 25, and this space is formed as a second space 17a.

The second blade part 26 is formed, for example, in a downward convex curved shape, and is provided as an other blade part for performing excision or the like of an affected area of a patient. The second blade part 26 protrudes forward from a position closer to the second opening pulley 24 side in a direction in which the second opening pulley 24 and the second closing pulley 25 are aligned.

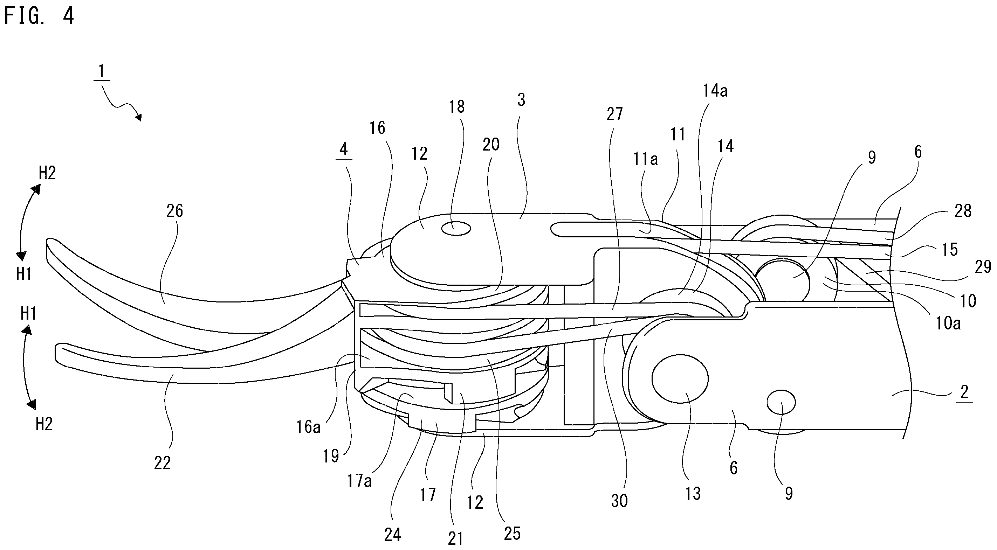

By rotating the first jaw 16 and the second jaw 17 in a closing direction (H1 shown in FIG. 4) with the rotating shaft 18 as a fulcrum, the first blade part 22 provided as one blade part and the second blade part 26 provided as the other blade part are moved in directions toward each other. By rotating the first jaw 16 and the second jaw 17 in the closing direction, for example, excision or the like of an affected area of a patient is performed.

On the other hand, by rotating the first jaw 16 and the second jaw 17 in an opening direction (H2 shown in FIG. 4) with the rotating shaft 18 as a fulcrum, the first blade part 22 and the second blade part 26 are moved in a direction away from each other. By rotating the first jaw 16 and the second jaw 17 in the opening direction about the rotating shaft 18 as a fulcrum, a surgical procedure or the like such as widening a size of a hole formed in a tissue such as a blood vessel is performed, for example, in a state in which the first blade part 22 and the second blade part 26 are inserted therein.

In the end effector 4, the rotating shaft 18 passes through the first opening pulley 20, the first closing pulley 21, the second opening pulley 24, and the second closing pulley 25, the second closing pulley 25 is positioned in the first space 16a between the first opening pulley 20 and the first closing pulley 21, and the first closing pulley 21 is positioned in the second space 17a between the second opening pulley 24 and the second closing pulley 25. Therefore, in the end effector 4, the first opening pulley 20, the second closing pulley 25, the first closing pulley 21, and the second opening pulley 24 are arranged in order in the axial direction of the rotating shaft 18.

One end portion of a first opening wire 27 is connected to the first opening pulley 20 of the first jaw 16. The first opening wire 27 is wound around the pulley portion 14a of the third auxiliary pulley 14 and the pulley portion 10a of the second auxiliary pulley 10 in order from the first opening pulley 20 side.

One end portion of a first closing wire 28 is connected to the first closing pulley 21 of the first jaw 16. The first closing wire 28 is wound around the pulley portion 14b of the third auxiliary pulley 14, the pulley portion 10b of the second auxiliary pulley 10, and the first auxiliary pulley 8 in order from the first closing pulley 21 side.

One end portion of a second opening wire 29 is connected to the second opening pulley 24 of the second jaw 17. The second opening wire 29 is wound around the pulley portion 14a of the third auxiliary pulley 14 and the pulley portion 10a of the second auxiliary pulley 10 in order from the second opening pulley 24 side.

One end portion of a second closing wire 30 is connected to the second closing pulley 25 of the second jaw 17. The second closing wire 30 is wound around the pulley portion 14b of the third auxiliary pulley 14, the pulley portion 10b of the second auxiliary pulley 10, and the first auxiliary pulley 8 in order from the second closing pulley 25 side.

### <Operation of the Surgical Instrument According to the First Embodiment>

The operation of the surgical instrument 1 will be described below (see FIG. 4 to FIG. 7).

When the first opening wire 27 connected to the first opening pulley 20 is pulled, the first jaw 16 is rotated in one direction about the rotating shaft 18 as a fulcrum, and the first jaw 16 is operated in the opening direction (see FIG. 4). At this time, the first closing wire 28 connected to the first closing pulley 21 is pulled back in the direction of winding around the first closing pulley 21 as the first jaw 16 is operated in the opening direction.

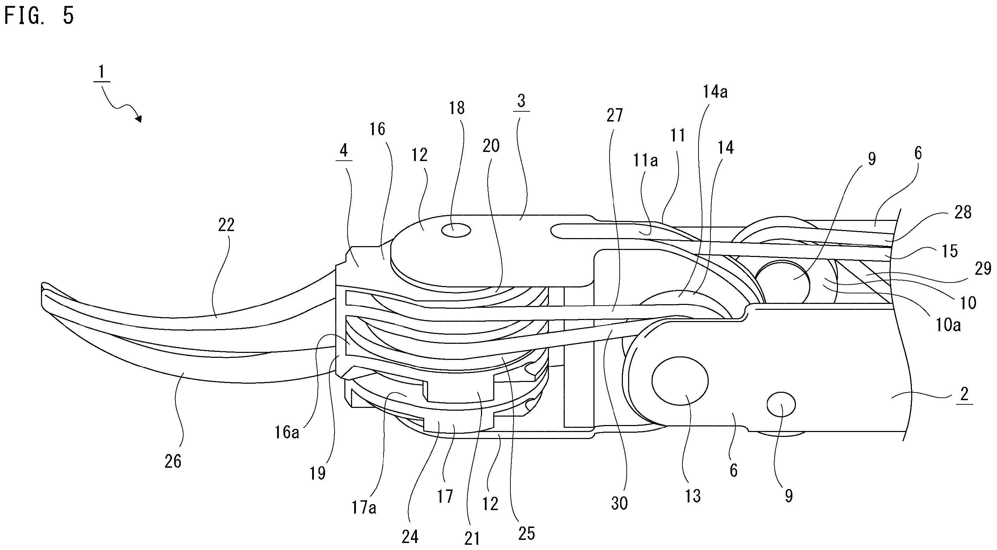

Conversely, when the first closing wire 28 connected to the first closing pulley 21 is pulled, the first jaw 16 is rotated about the rotating shaft 18 as a fulcrum in the other direction, and the first jaw 16 is operated in the closing direction (see FIG. 5). At this time, the first opening wire 27 connected to the first opening pulley 20 is pulled back in the direction of winding around the first opening pulley 20 as the first jaw 16 is operated in the closing direction.

On the other hand, when the second opening wire 29 connected to the second opening pulley 24 is pulled, the second jaw 17 is rotated in one direction with the rotating shaft 18 as a fulcrum, and the second jaw 17 is operated in the opening direction (see FIG. 4). At this time, the second closing wire 30 connected to the second closing pulley 25 is pulled back in the direction of winding around the second closing pulley 25 as the second jaw 17 is operated in the opening direction.

Conversely, when the second closing wire 30 connected to the second closing pulley 25 is pulled, the second jaw 17 is rotated in the other direction with the rotating shaft 18 as a fulcrum, and the second jaw 17 is operated in the closing direction (see FIG. 5). At this time, the second opening wire 29 connected to the second opening pulley 24 is pulled back in the direction of winding around the second opening pulley 24 as the second jaw 17 is operated in the closing direction.

In the surgical instrument 1, the above-described opening operation of the first jaw 16 and opening operation of the second jaw 17 are performed simultaneously, and by the opening operation, the first blade part 22 and the second blade part 26 are moved in directions separating from each other; for example, a surgical procedure or the like is performed to widen the size of a hole formed in a tissue such as a blood vessel. In addition, the above-described closing operation of the first jaw 16 and closing operation of the second jaw 17 are performed simultaneously, and by the closing operation, the first blade part 22 and the second blade part 26 are moved in directions toward each other; for example, excision or the like of the patient's affected area is performed.

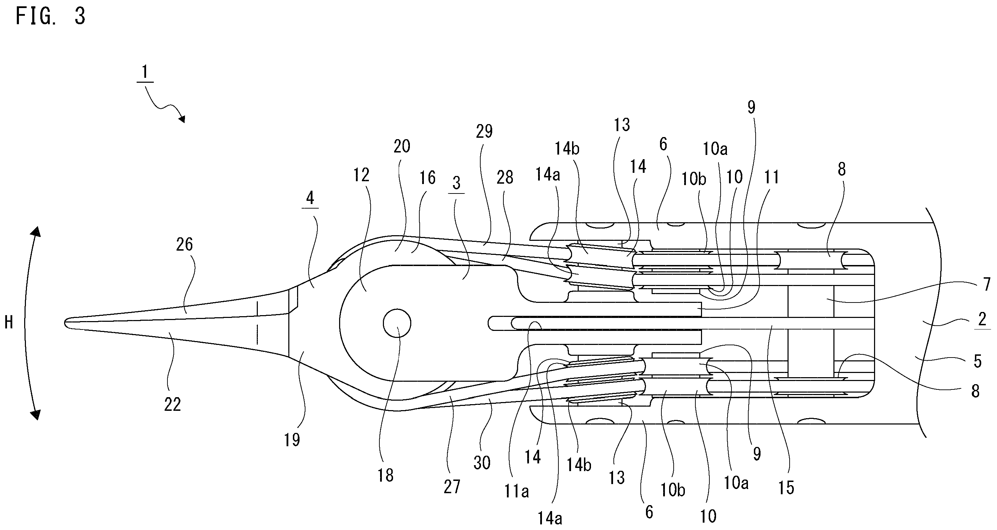

In the end effector 4, the first opening pulley 20 is positioned closer to the first blade part 22 side than the second blade part 26 with respect to the first closing pulley 21 in the direction orthogonal to the opening and closing direction (H shown in FIG. 3) (vertical direction).

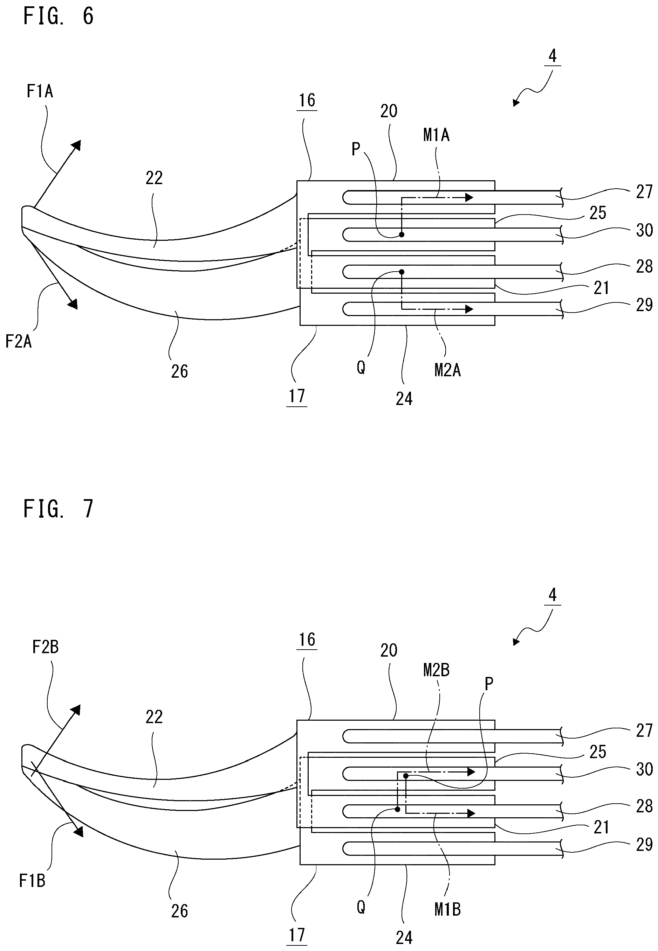

Therefore, when the first opening wire 27 connected to the first opening pulley 20 is pulled to operate the first jaw 16 in the opening direction, a rotational moment M1A, with the midpoint P in a direction in which the first opening pulley 20 and the first closing pulley 21 are arranged as a reference, causes a force F1A to be applied to the first blade part 22 in a direction away from the second blade part 26 (see FIG. 6).

On the other hand, in the end effector 4, the first closing pulley 21 is positioned closer to the second blade part 26 side than the first blade part 22 with respect to the first opening pulley 20 in a direction orthogonal to the opening and closing direction.

Therefore, when the first closing wire 28 connected to the first closing pulley 21 is pulled and the first jaw 16 is operated in the closing direction, a rotational moment M1B with the midpoint P as a reference causes a force F1B to be applied to the first blade part 22 in the direction of contact with the second blade part 26 (see FIG. 7).

In addition, in the end effector 4, the second opening pulley 24 is positioned closer to the second blade part 26 side than the first blade part 22 with respect to the second closing pulley 25 in the direction orthogonal to the opening and closing direction.

Therefore, when the second opening wire 29 connected to the second opening pulley 24 is pulled to operate the second jaw 17 in the opening direction, a rotational moment M2A, with the midpoint Q in a direction in which the second opening pulley 24 and the second closing pulley 25 are arranged as a reference, causes a force F2A to be applied to the second blade part 26 in a direction away from the first blade part 22 (see FIG. 6).

On the other hand, in the end effector 4, the second closing pulley 25 is positioned closer to the first blade part 22 side than the second blade part 26 with respect to the second opening pulley 24 in a direction orthogonal to the opening and closing direction.

Therefore, when the second closing wire 30 connected to the second closing pulley 25 is pulled and the second jaw 17 is operated in the closing direction, a rotational moment M2B, with the midpoint Q as a reference, causes a force F2B to be applied to the second blade part 26 in the direction of contact with the first blade part 22 (see FIG. 7).

When the first jaw 16 and the second jaw 17 are operated in the opening direction and the first blade part 22 and the second blade part 26 are moved in directions separating from each other in this way, the rotational moment M1A and the rotational moment M2A cause forces to be respectively applied to the first blade part 22 and the second blade part 26 in directions away from each other and orthogonal to the direction of the opening motion (see FIG. 6).

Therefore, when the first blade part 22 and the second blade part 26 are moved from a closed state (see FIG. 5) to an open state (see FIG. 4), a frictional force between the first blade part 22 and the second blade part 26 is reduced, it becomes possible to perform the opening operation of the first jaw 16 and the second jaw 17 with a small driving force, and it is possible to ensure a stable and smooth opening operation of the end effector 4.

In particular, when the first blade part 22 and the second blade part 26 are inserted into a tissue such as a blood vessel and a surgical procedure or the like such as widening a size of a hole formed in the tissue is performed, it is possible to perform the opening operation at a low speed or at a constant speed with a small driving force, and it is possible to perform the operation with less burden on the patient.

In addition, when the first jaw 16 and the second jaw 17 are operated in the closing direction and the first blade part 22 and the second blade part 26 are moved in directions toward each other, the rotation moment M1B and the rotation moment M2B cause forces to be respectively applied to the first blade part 22 and the second blade part 26 in directions to contact with each other and orthogonal to the direction of the closing operation (see FIG. 7).

Therefore, when the first blade part 22 and the second blade part 26 are moved from the open state (see FIG. 4) to the closed state (see FIG. 5), it becomes possible for the first blade part 22 and the second blade part 26 to generate a good sharpness against shear resistance, and it is possible to ensure a stable and smooth closing operation of the end effector 4.

In particular, in a case where it is desired to excise an affected area of a patient by using the first blade part 22 and the second blade part 26 or the like, it becomes possible to perform the excision or the like without causing significant damage to the tissue, and thus surgery may be performed with less burden on the patient.

Note that in the surgical instrument 1, the support body 3 is rotatably connected to the base body 2 with the fulcrum shaft 13 as a fulcrum, and thus by pulling the support body wire 15, the end effector 4 is rotated together with the support body 3 with respect to the base body 2 with the fulcrum shaft 13 as a fulcrum.

In addition, the support body 3 is rotatably connected to the base body 2 with the fulcrum shaft 13 as a fulcrum, and the axial direction of the rotating shaft 18 that serves as a rotation fulcrum for the end effector 4 to rotate with respect to the support body 3 and the axial direction of the fulcrum shaft 13 are orthogonal to each other.

Therefore, since it is possible to operate the end effector 4 in two orthogonal directions, it is possible to perform surgery with high positional accuracy and a high degree of freedom of movement.

Furthermore, in the surgical instrument 1, the second closing pulley 25 is positioned between the first opening pulley 20 and the first closing pulley 21, and the first closing pulley 21 is positioned between the second opening pulley 24 and the second closing pulley 25.

Therefore, since the pulleys of the first jaw 16 and the pulleys of the second jaw 17 are alternately positioned, the space for arranging the four pulleys in the axial direction of the rotating shaft 18 can be reduced, and it becomes possible to position the two pulleys of the first jaw 16 and the two pulleys of the second jaw 17 separately. As a result, the size of the end effector 4 can be reduced, and in the opening and closing operations, it is possible to apply forces F1A and F2A to the first blade part 22 and the second blade part 26 in directions away from each other and apply forces F1B and F2B to the first blade part 22 and the second blade part 26 in directions to contact each other.

Furthermore, the first opening pulley 20, the second closing pulley 25, the first closing pulley 21, and the second opening pulley 24 are positioned in order in the axial direction of the rotating shaft 18, and in the direction orthogonal to the opening and closing direction (vertical direction), the first opening pulley 20 is positioned on the first blade part 22 side, and the second opening pulley 24 is positioned on the second blade part 26 side.

Therefore, since the first opening pulley 20 and the second opening pulley 24 are positioned on both sides of the second closing pulley 25 and the first closing pulley 21, and in particular, configuration becomes such that it is possible to increase the moment in the opening operation, and thus, a moderate force may be applied to the first blade part 22 and the second blade part 26 in the direction of contact in the closing operation, and a large force may be applied to the first blade part 22 and the second blade part 26 in the separating direction in the opening operation.

In addition, the first jaw 16 is provided with the first base portion 19, and the first opening pulley 20 and the first closing pulley 21 protrude in the same direction from both end portions of the first base portion 19, and the second jaw 17 is provided with the second base portion 23, and the second opening pulley 24 and the second closing pulley 25 protrude in the same direction from both end portions of the second base portion 23.

Therefore, since both the first jaw 16 and the second jaw 17 are formed in a bifurcated shape, it becomes possible to alternately position the pulleys of the first jaw 16 and the pulleys of the second jaw 17 with a simple structure, and it is possible to simplify the structure and reduce the manufacturing cost.

### <Configuration of Surgical Instrument According to a Second Embodiment>

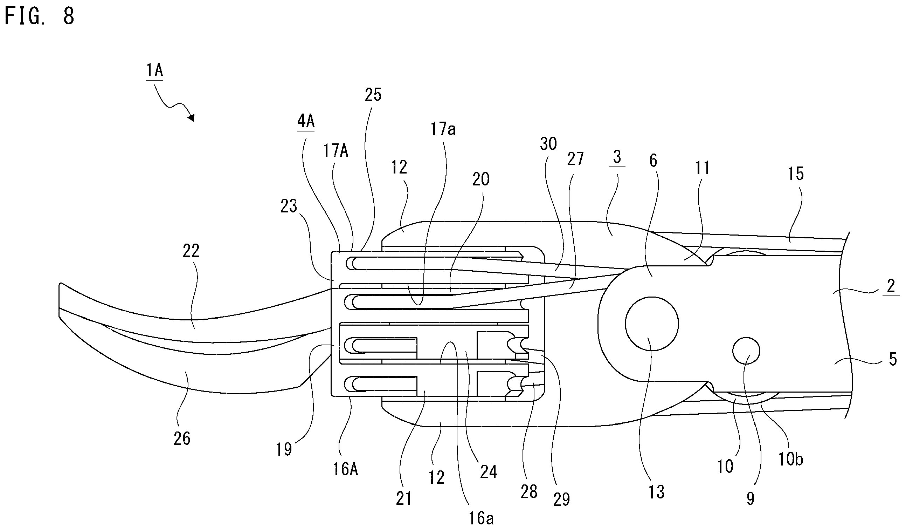

Next, the configuration of the surgical instrument 1A according to a second embodiment will be described (see FIG. 8 to FIG. 11).

Note that since the surgical instrument 1A shown below differs from the surgical instrument 1 described above only in the configuration of the end effector, only the parts that differ from the surgical instrument 1 will be described in detail, and for the other parts, the same reference numerals will be given to parts that are the same as parts in the surgical instrument 1, and descriptions thereof will be omitted. In addition, in the surgical instrument 1A described below, the end effector is indicated as an end effector 4A, and the first jaw 16 and the second jaw 17 are indicated as a first jaw 16A and a second jaw 17A, respectively.

The surgical instrument 1A is attachable to and detachable from the mounting portion 57a of the second connecting arm 57. The surgical instrument 1A has a base body 2, a support body 3 and an end effector 4A.

The end effector 4A is composed of a first jaw 16A and a second jaw 17A. The first jaw 16A is, for example, a portion that constitutes the left side (L side) scissor, and the second jaw 17A is, for example, a portion that constitutes the right side (R side) scissor.

The end effector 4A is rotatably supported by the support body 3 with the rotating shaft 18 as a fulcrum.

The first jaw 16A includes a first base portion 19 formed in a plate shape facing in a substantially front-rear direction, a first opening pulley 20 protruding rearward from an upper end portion of the first base portion 19, a first closing pulley 21 protruding rearward from a lower end portion of the first base portion 19, and a first blade part 22 protruding forward from an upper end portion of the first base portion 19.

In the first jaw 16A, the first base portion 19, the first opening pulley 20, the first closing pulley 21, and the first blade part 22 are integrally formed, for example.

As described above, in the first jaw 16A, the first base portion 19, the first opening pulley 20, the first closing pulley 21, and the first blade part 22 are integrally formed, and thus the first jaw 16A may be formed of one member, and the manufacturing cost of the surgical instrument 1A may be reduced.

In the first jaw 16A, by passing the rotating shaft 18 through central portions of the first opening pulley 20 and the first closing pulley 21, the first jaw 16A is rotatable with respect to the support body 3 with the rotating shaft 18 as a fulcrum.

As described above, the axial direction of the rotating shaft 18 is vertically oriented, and the support body 3 is rotated with respect to the base body 2 about the fulcrum shaft 13 as a fulcrum, the axial direction of which is oriented in the left-right direction. Therefore, the direction of rotation of the first jaw 16A with respect to the support body 3 and the direction of rotation of the support body 3 with respect to the base body 2 are orthogonal to each other.

In the first jaw 16A, the first opening pulley 20 and the first closing pulley 21 protrude rearward from both the upper and lower end portions of the first base portion 19, and thus the first jaw 16A has a space between the first opening pulley 20 and the first closing pulley 21, and this space is formed as a first space 16a.

The first blade part 22 is formed, for example, in a downward convex curved shape, and is provided as one blade part for performing excision or the like of an affected area of a patient. The first blade part 22 protrudes forward from the same portion of the first base portion 19 that the first opening pulley 20 protrudes.

The second jaw 17A includes a second base portion 23 formed in a plate shape facing in a substantially front-rear direction, a second opening pulley 24 protruding rearward from a lower end portion of the second base portion 23, a second closing pulley 25 protruding rearward from an upper end portion of the second base portion 23, and a second blade part 26 protruding forward from a lower end portion of the second base portion 23.

In the second jaw 17A, the second base portion 23, the second opening pulley 24, the second closing pulley 25, and the second blade part 26 are integrally formed, for example.

As described above, in the second jaw 17A, the second base portion 23, the second opening pulley 24, the second closing pulley 25, and the second blade part 26 are integrally formed, and thus the jaw 17A may be formed of one member, and the manufacturing cost of the surgical instrument 1A may be reduced.

In the second jaw 17A, by passing the rotating shaft 18 through central portions of the second opening pulley 24 and the second closing pulley 25, the second jaw 17A is rotatable with respect to the support body 3 with the rotating shaft 18 as a fulcrum.

As described above, the axial direction of the rotating shaft 18 is vertically oriented, and the support body 3 is rotated with respect to the base body 2 about the fulcrum shaft 13 as a fulcrum, the axial direction of which is oriented in the left-right direction. Therefore, the direction of rotation of the second jaw 17A with respect to the support body 3 and the direction of rotation of the support body 3 with respect to the base body 2 are orthogonal to each other.

In the second jaw 17A, the second closing pulley 25 and the second opening pulley 24 protrude rearward from both the upper and lower end portions of the second base portion 23, and thus the second jaw 17A has a space between the second opening pulley 24 and the second closing pulley 25, and this space is formed as a second space 17a.

The second blade part 26 is formed, for example, in a downward convex curved shape, and is provided as an other blade part for performing excision or the like of an affected area of a patient. The second blade part 26 protrudes forward from the same portion of the second base portion 23 that the second opening pulley 24 protrudes.

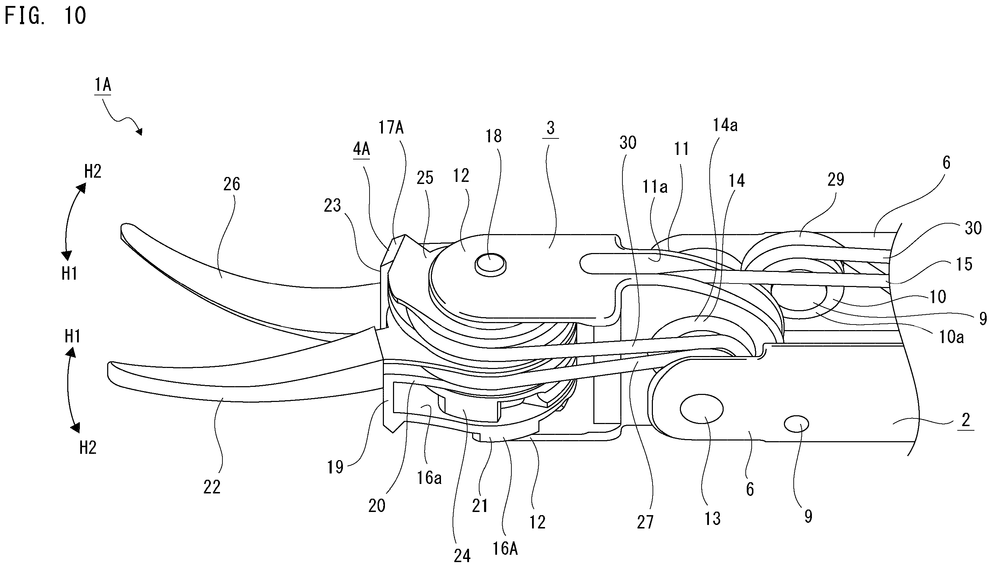

By rotating the first jaw 16A and the second jaw 17A in a closing direction (H1 shown in FIG. 10) with the rotating shaft 18 as a fulcrum, the first blade part 22 provided as one blade part and the second blade part 26 provided as the other blade part are moved in directions toward each other. By rotating the first jaw 16A and the second jaw 17A in the closing direction, for example, excision or the like of an affected area of a patient is performed.

On the other hand, by rotating the first jaw 16A and the second jaw 17A in an opening direction (H2 shown in FIG. 10) with the rotating shaft 18 as a fulcrum, the first blade part 22 provided as one blade part and the second blade part 26 provided as the other blade part are moved in directions separating from each other. By rotating the first jaw 16A and the second jaw 17A in the opening direction, for example, a surgical procedure or the like such as widening a size of a hole formed in a tissue such as a blood vessel is performed, for example, in a state in which the first blade part 22 and the second blade part 26 are inserted therein.

In the end effector 4A, the rotating shaft 18 passes through the first opening pulley 20, the first closing pulley 21, the second opening pulley 24, and the second closing pulley 25, the second opening pulley 24 is positioned in the first space 16a between the first opening pulley 20 and the first closing pulley 21, and the first opening pulley 20 is positioned in the second space 17a between the second opening pulley 24 and the second closing pulley 25. Therefore, in the end effector 4A, the second closing pulley 25, the first opening pulley 20, the second opening pulley 24, and the first closing pulley 21 are arranged in order in the axial direction of the rotating shaft 18.

One end portion of the first opening wire 27 is connected to the first opening pulley 20 of the first jaw 16A. The first opening wire 27 is wound around the pulley portion 14b of the third auxiliary pulley 14, the pulley portion 10b of the second auxiliary pulley 10, and the first auxiliary pulley 8 in order from the first opening pulley 20 side.

One end portion of the first closing wire 28 is connected to the first closing pulley 21 of the first jaw 16A. The first closing wire 28 is wound around the pulley portion 14a of the third auxiliary pulley 14 and the pulley portion 10a of the second auxiliary pulley 10 in order from the first closing pulley 21 side.

One end portion of a second opening wire 29 is connected to the second opening pulley 24 of the second jaw 17A. The second opening wire 29 is wound around the pulley portion 14b of the third auxiliary pulley 14, the pulley portion 10b of the second auxiliary pulley 10, and the first auxiliary pulley 8 in order from the second opening pulley 24 side.

One end portion of a second closing wire 30 is connected to the second closing pulley 25 of the second jaw 17A. The second closing wire 30 is wound around the pulley portion 14a of the third auxiliary pulley 14 and the pulley portion 10a of the second auxiliary pulley 10 in order from the second closing pulley 25 side.

### <Operation of the Surgical Instrument According to the Second Embodiment>

The operation of the surgical instrument 1A will be described below (see FIG. 10 to FIG. 13).

When the first opening wire 27 connected to the first opening pulley 20 is pulled, the first jaw 16A is rotated in one direction with the rotating shaft 18 as a fulcrum, and the first jaw 16A is operated in the opening direction (see FIG. 10). At this time, the first closing wire 28 connected to the first closing pulley 21 is pulled back in the direction of winding around the first closing pulley 21 as the first jaw 16A is operated in the opening direction.

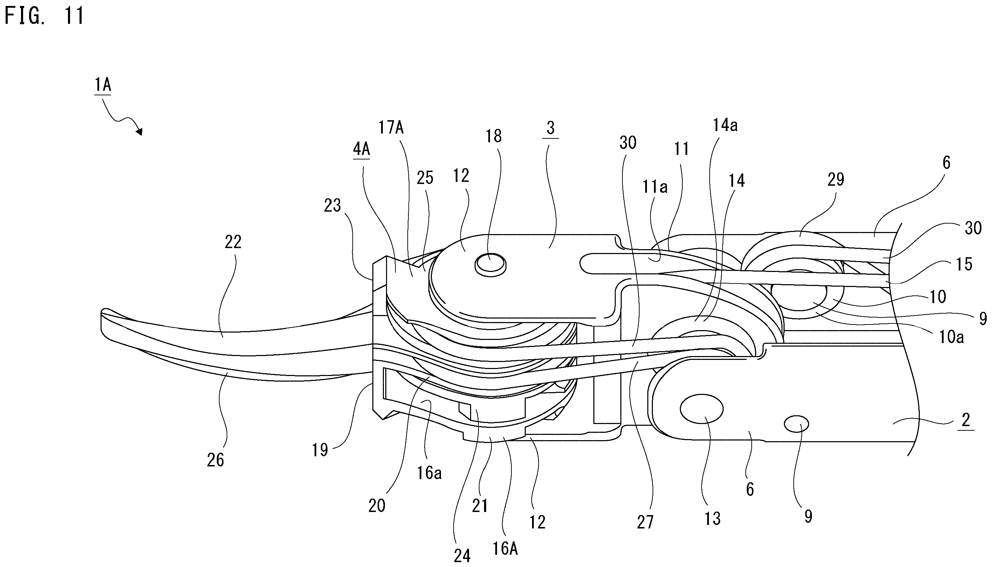

Conversely, when the first closing wire 28 connected to the first closing pulley 21 is pulled, the first jaw 16A is rotated about the rotating shaft 18 as a fulcrum in the other direction, and the first jaw 16A is operated in the closing direction (see FIG. 11). At this time, the first opening wire 27 connected to the first opening pulley 20 is pulled back in the direction of winding around the first opening pulley 20 as the first jaw 16A is operated in the closing direction.

On the other hand, when the second opening wire 29 connected to the second opening pulley 24 is pulled, the second jaw 17A is rotated in one direction with the rotating shaft 18 as a fulcrum, and the second jaw 17A is operated in the opening direction (see FIG. 10). At this time, the second closing wire 30 connected to the second closing pulley 25 is pulled back in the direction of winding around the second closing pulley 25 as the second jaw 17A is operated in the opening direction.

Conversely, when the second closing wire 30 connected to the second closing pulley 25 is pulled, the second jaw 17A is rotated in the other direction with the rotating shaft 18 as a fulcrum, and the second jaw 17A is operated in the closing direction (see FIG. 11). At this time, the second opening wire 29 connected to the second opening pulley 24 is pulled back in the direction of winding around the second opening pulley 24 as the second jaw 17A is operated in the closing direction.

In the surgical instrument 1A, the above-described opening operation of the first jaw 16A and opening operation of the second jaw 17A are performed simultaneously, and by the opening operation, the first blade part 22 and the second blade part 26 are moved in directions separating from each other; for example, a surgical procedure or the like is performed to widen the size of a hole formed in a tissue such as a blood vessel. In addition, the above-described closing operation of the first jaw 16A and closing operation of the second jaw 17A are performed simultaneously, and by the closing operation, the first blade part 22 and the second blade part 26 are moved in directions toward each other; for example, excision or the like of the patient's affected area is performed.

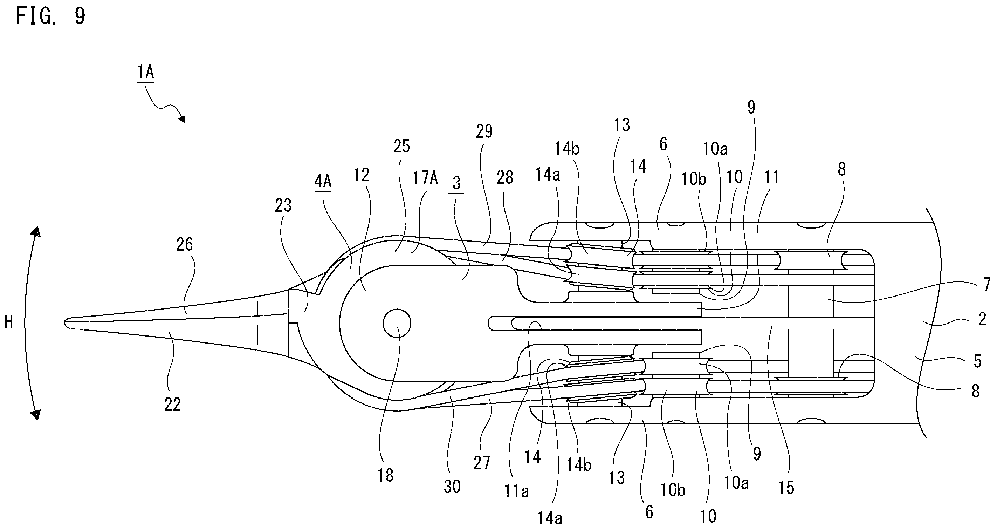

In the end effector 4A, the first opening pulley 20 is positioned closer to the first blade part 22 side than the second blade part 26 with respect to the first closing pulley 21 in the direction orthogonal to the opening and closing direction (H shown in FIG. 9) (vertical direction).

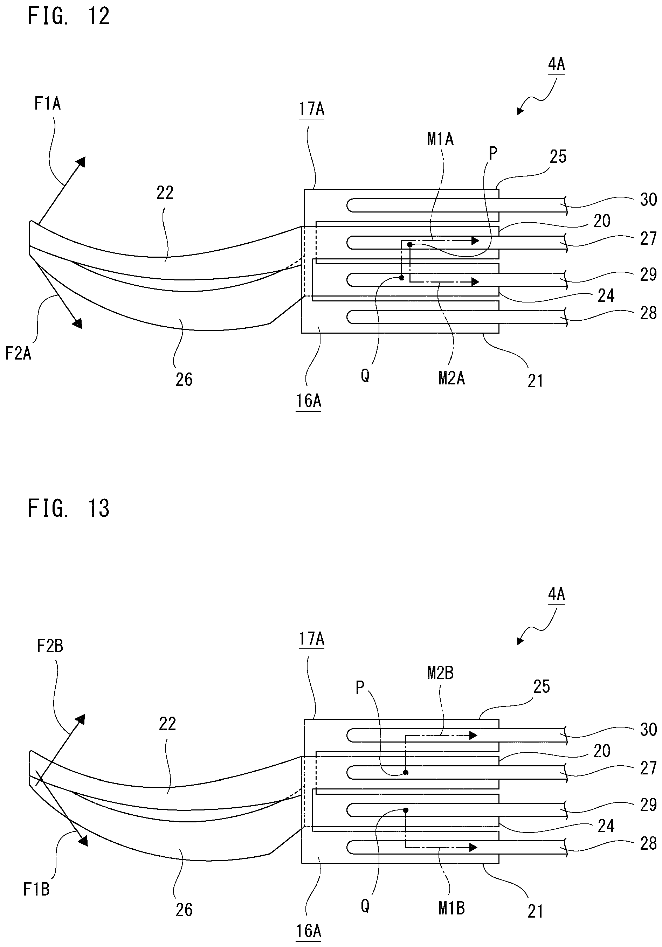

Therefore, when the first opening wire 27 connected to the first opening pulley 20 is pulled to operate the first jaw 16A in the opening direction, a rotational moment M1A, with the midpoint P in a direction in which the first opening pulley 20 and the first closing pulley 21 are arranged as a reference, causes a force F1A to be applied to the first blade part 22 in a direction away from the second blade part 26 (see FIG. 12).

On the other hand, in the end effector 4A, the first closing pulley 21 is positioned closer to the second blade part 26 side than the first blade part 22 with respect to the first opening pulley 20 in a direction orthogonal to the opening and closing direction.

Therefore, when the first closing wire 28 connected to the first closing pulley 21 is pulled and the first jaw 16A is operated in the closing direction, a rotational moment M1B with the midpoint P as a reference causes a force F1B to be applied to the first blade part 22 in the direction of contact with the second blade part 26 (see FIG. 13).

In addition, in the end effector 4A, the second opening pulley 24 is positioned closer to the second blade part 26 side than the first blade part 22 with respect to the second closing pulley 25 in the direction orthogonal to the opening and closing direction.

Therefore, when the second opening wire 29 connected to the second opening pulley 24 is pulled to operate the second jaw 17A in the opening direction, a rotational moment M2A, with the midpoint Q in a direction in which the second opening pulley 24 and the second closing pulley 25 are arranged as a reference, causes a force F2A to be applied to the second blade part 26 in a direction away from the first blade part 22 (see FIG. 12).

On the other hand, in the end effector 4A, the second closing pulley 25 is positioned closer to the first blade part 22 side than the second blade part 26 with respect to the second opening pulley 24 in a direction orthogonal to the opening and closing direction.

Therefore, when the second closing wire 30 connected to the second closing pulley 25 is pulled and the second jaw 17A is operated in the closing direction, a rotational moment M2B, with the midpoint Q as a reference, causes a force F2B to be applied to the second blade part 26 in the direction of contact with the first blade part 22 (see FIG. 13).

When the first jaw 16A and the second jaw 17A are operated in the opening direction and the first blade part 22 and the second blade part 26 are moved in directions separating from each other in this way, the rotational moment M1A and the rotational moment M2A cause forces to be respectively applied to the first blade part 22 and the second blade part 26 in directions separating from each other and orthogonal to the direction of the opening motion (see FIG. 12).

Therefore, when the first blade part 22 and the second blade part 26 are moved from a closed state (see FIG. 11) to an open state (see FIG. 10), a frictional force between the first blade part 22 and the second blade part 26 is reduced, it becomes possible to perform the opening operation of the first jaw 16A and the second jaw 17A with a small driving force, and it is possible to ensure a stable and smooth opening operation of the end effector 4A.

In particular, when the first blade part 22 and the second blade part 26 are inserted into a tissue such as a blood vessel and a surgical procedure or the like such as widening a size of a hole formed in the tissue is performed, it is possible to perform the opening operation at a low speed or at a constant speed with a small driving force, and it is possible to perform the operation with less burden on the patient.

In addition, when the first jaw 16A and the second jaw 17A are operated in the closing direction and the first blade part 22 and the second blade part 26 are moved in directions toward each other, the rotation moment M1B and the rotation moment M2B cause forces to be respectively applied to the first blade part 22 and the second blade part 26 in directions to contact with each other and orthogonal to the direction of the closing operation (see FIG. 13).

Therefore, when the first blade part 22 and the second blade part 26 are moved from the open state (see FIG. 10) to the closed state (see FIG. 11), it becomes possible for the first blade part 22 and the second blade part 26 to generate a good sharpness against shear resistance, and it is possible to ensure a stable and smooth closing operation of the end effector 4A.

In particular, in a case where it is desired to excise an affected area of a patient by using the first blade part 22 and the second blade part 26 or the like, it becomes possible to perform the excision or the like without causing significant damage to the tissue, and thus surgery may be performed with less burden on the patient.

Note that in the surgical instrument 1A, the support body 3 is rotatably connected to the base body 2 with the fulcrum shaft 13 as the fulcrum, and thus by pulling the support body wire 15, the end effector 4A is rotated together with the support body 3 with respect to the base body 2 with the fulcrum shaft 13 as the fulcrum.

In addition, the support body 3 is rotatably connected to the base body 2 with the fulcrum shaft 13 as a fulcrum, and the axial direction of the rotating shaft 18 that serves as a rotation fulcrum for the end effector 4A to rotate with respect to the support body 3 and the axial direction of the fulcrum shaft 13 are orthogonal to each other.

Therefore, since it is possible to operate the end effector 4Ain two orthogonal directions, it is possible to perform surgery with high positional accuracy and a high degree of freedom of movement.

Furthermore, in the surgical instrument 1A, the second opening pulley 24 is positioned between the first opening pulley 20 and the first closing pulley 21, and the first opening pulley 20 is positioned between the second opening pulley 24 and the second closing pulley 25.

Therefore, since the pulleys of the first jaw 16A and the pulleys of the second jaw 17A are alternately positioned, the space for arranging the four pulleys in the axial direction of the rotating shaft 18 can be reduced, and it becomes possible to position the two pulleys of the first jaw 16A and the two pulleys of the second jaw 17A separately. As a result, the size of the end effector 4A can be reduced, and in the opening and closing operations, it is possible to apply forces F1A and F2A to the first blade part 22 and the second blade part 26 in directions away from each other and apply forces F1B and F2B to the first blade part 22 and the second blade part 26 in directions to contact each other.

Furthermore, the second closing pulley 25, the first opening pulley 20, the second opening pulley 24, and the first closing pulley 21 are positioned in order in the axial direction of the rotating shaft 18, and the second closing pulley 25 is positioned on the first blade part 22 side and the first closing pulley 21 is positioned on the second blade part 26 side in the direction orthogonal to the opening and closing direction (vertical direction).

Therefore, since the second closing pulley 25 and the first closing pulley 21 are positioned on both sides of the first opening pulley 20 and the second opening pulley 24, and in particular, configuration becomes such that it is possible to increase the moment in the closing operation, and thus, a moderate force may be applied to the first blade part 22 and the second blade part 26 in the separating direction in the opening operation, and a large force may be applied to the first blade part 22 and the second blade part 26 in the direction of contact in the closing operation.

In addition, together with the first jaw 16A being provided with the first base portion 19, the first opening pulley 20 and the first closing pulley 21 protrude in the same direction from both end portions of the first base portion 19, and together with the second jaw 17A being provided with the second base portion 23, the second opening pulley 24 and the second closing pulley 25 protrude in the same direction from both end portions of the second base portion 23.

Therefore, since both the first jaw 16A and the second jaw 17A are formed in a bifurcated shape, it becomes possible to alternately position the pulleys of the first jaw 16A and the pulleys of the second jaw 17A with a simple structure, and it is possible to simplify the structure and reduce the manufacturing cost.

### <Summary>

As described above, in the surgical instrument 1 and the surgical instrument 1A, the first blade part 22 and the second blade part 26 are operated in opening directions separating from each other by pulling the first opening wire 27 and the second opening wire 29, respectively, and are operated in closing directions approaching each other by pulling the first closing wire 28 and the second closing wire 30, respectively. In addition, the first opening pulley 20 is positioned closer to the first blade part 22 side than the second blade part 26 with respect to the first closing pulley 21 in the direction orthogonal to the opening and closing direction, and the second opening pulley 24 is positioned closer to the first blade part 22 side than the second blade part 26 with respect to the second closing pulley 25 in the direction orthogonal to the opening and closing direction.

Therefore, moments M1A and M2A that are respectively generated in the opening operation of the first jaw 16 and the second jaw 17 apply forces to the first blade part 22 and the second blade part 26 in directions separating from each other and in directions orthogonal to the opening and closing direction, and moments M1B and M2B that are respectively generated in the closing operation of the first jaw 16 and the second jaw 17 apply forces to the first blade part 22 and the second blade part 26 in directions to contact with each other and in directions orthogonal to the opening and closing direction, and thus a stable and highly accurate operating state of the end effectors 4 and 4A can be ensured.

### REFERENCE SIGNS LIST

50 Surgical robot
54 First support arm (robot arm)
55 Second support arm (robot arm)
56 First connecting arm (robot arm)
57 Second connecting arm (robot arm)
1 Surgical instrument
2 Base body
3 Support body
4 End effector
13 Fulcrum shaft
16 First jaw
17 Second jaw
18 Rotating shaft
19 First base portion
20 First opening pulley
21 First closing pulley
22 First blade part
23 Second base portion
24 Second opening pulley
25 Second closing pulley
26 Second blade part
27 First opening wire
28 First closing wire
29 Second opening wire
30 Second closing wire
1A Surgical instrument
4A End effector
16A First jaw
17A Second jaw

## Claims

1. A surgical instrument (1) attached to a surgical robot arm, the surgical instrument (1) comprising:
a base body (2) detachable from the robot arm;
a support body (3) connected to the base body (2); and
an end effector (4) including a first jaw (16) and a second jaw (17) that are each rotatably supported by the support body (3) with a rotating shaft (18) as a fulcrum; wherein
the first jaw (16) includes a first opening pulley (20) to which a first opening wire (27) is connected, a first closing pulley (21) to which a first closing wire (28) is connected, and a first blade part (22) operated in an opening and closing direction, wherein the first jaw (16) is provided with a first base portion (19), and the first opening pulley (20) and the first closing pulley (21) protrude in a same direction from both end portions of the first base portion (19);
the second jaw (17) includes a second opening pulley (24) to which a second opening wire (29) is connected, a second closing pulley (25) to which a second closing wire (30) is connected, and a second blade part (26) operated in the opening and closing direction, wherein the second jaw (17) is provided with a second base portion (23), and the second opening pulley (24) and the second closing pulley (25) protrude in a same direction from both end portions of the second base portion (23);
the first opening pulley (20) and the first closing pulley (21) are supported by the rotating shaft (18) in a state of being separated from each other in an axial direction of the rotating shaft (18);
the second opening pulley (24) and the second closing pulley (25) are supported by the rotating shaft (18) in a state of being separated from each other in the axial direction of the rotating shaft (18);
the first blade part (22) and the second blade part (26) are operated in opening directions separating from each other by pulling the first opening wire (27) and the second opening wire (29), respectively, and are operated in closing directions approaching each other by pulling the first closing wire (28) and the second closing wire (30), respectively;
the first blade part (22) protrudes from the first base portion (19) in a direction opposite to the first opening pulley (20) and the first closing pulley (21) and the first blade part (22) protrudes from a position between the first opening pulley (20) and the first closing pulley (21), and closer to the first opening pulley (20) so that the first opening pulley (20) is positioned closer to the first blade part side than the second blade part (26) with respect to the first closing pulley (21) in a direction orthogonal to the opening and closing direction;
the second blade part (26) protrudes from the second base portion (23) in a direction opposite to the second opening pulley (24) and the second closing pulley (25), and the second blade part (26) protrudes from a position between the second opening pulley (24) and the second closing pulley (25), and closer to the second opening pulley (24) so that the second opening pulley (24) is positioned closer to the second blade part side than the first blade part (22) with respect to the second closing pulley (25) in a direction orthogonal to the opening and closing direction,
the first blade part (22) and the second blade part (26) are arranged side by side in a direction orthogonal to the opening and closing direction;
the second opening pulley (24) or the second closing pulley (25) is positioned between the first opening pulley (20) and the first closing pulley (21); and
the first opening pulley (20) or the first closing pulley (21) is positioned between the second opening pulley (24) and the second closing pulley (25), and **characterized in that**
the first opening pulley (20), the second closing pulley (25), the first closing pulley (21), and the second opening pulley (24) are positioned in order in the axial direction of the rotating shaft (18); and
in a direction orthogonal to the opening and closing direction, the first opening pulley (20) is positioned on the first blade part side, and the second opening pulley (24) is positioned on the second blade part side.

2. The surgical instrument (1) according to claim 1, wherein the first base portion (19), the first blade part (22), the first opening pulley (20), and the first closing pulley (21) are integrally formed; and
the second base portion (23), the second blade part (26), the second opening pulley (24), and the second closing pulley (25) are integrally formed.

3. The surgical instrument (1) according to any one of claim 1 or claim 2, wherein the support body (3) is rotatably connected to the base body (2) with a fulcrum shaft (13) as a fulcrum; and
the axial direction of the rotating shaft (18) and the axial direction of the fulcrum shaft (13) are orthogonal to each other.

## Patentansprüche

1. Chirurgisches Instrument (1), das an einem chirurgischen Roboterarm befestigt ist, wobei das chirurgische Instrument (1) enthält:
einen Basiskörper (2), der vom Roboterarm abnehmbar ist;
einen Haltekörper (3), der mit dem Basiskörper verbunden ist (2); und
einen Endeffektor (4) mit einer ersten Backe (16) und einer zweiten Backe (17), die jeweils drehbar vom Haltekörper (3) mit einer Drehwelle (18) als ein Drehpunkt gehalten werden; wobei
die erste Backe (16) eine erste Öffnungsrolle (20), mit der ein erster Öffnungsdraht (27) verbunden ist, eine erste Schließrolle (21), mit der ein erster Schließdraht (28) verbunden ist, und einen ersten Klingenteil (22) einschließt, der in einer Öffnungs- und Schließrichtung betrieben wird, wobei die erste Backe (16) mit einem ersten Basisabschnitt (19) bereitgestellt ist, und die erste Öffnungsrolle (20) und die erste Schließrolle (21) in eine gleiche Richtung aus beiden Endabschnitten des ersten Basisabschnitts (19) vorspringen;
die zweite Backe (17) eine zweite Öffnungsrolle (24), mit der ein zweiter Öffnungsdraht (29) verbunden ist, eine zweite Schließrolle (25), mit der ein zweiter Schließdraht (30) verbunden ist, und einen zweiten Klingenteil (26) einschließt, der in der Öffnungs- und Schließrichtung betrieben wird, wobei die zweite Backe (17) mit einem zweiten Basisabschnitt (23) bereitgestellt ist, und die zweite Öffnungsrolle (24) und die zweite Schließrolle (25) in eine gleiche Richtung aus beiden Endabschnitten des zweiten Basisabschnitts (23) vorspringen;
die erste Öffnungsrolle (20) und die erste Schließrolle (21) von der Drehwelle (18) in einem Zustand des voneinander Getrenntseins in einer axialen Richtung der Drehwelle (18) gehalten werden;
die zweite Öffnungsrolle (24) und die zweite Schließrolle (25) von der Drehwelle (18) in einem Zustand des voneinander Getrenntseins in einer axialen Richtung der Drehwelle (18) gehalten werden;
der erste Klingenteil (22) und der zweite Klingenteil (26) jeweils in Öffnungsrichtungen, sich voneinander trennend, durch Ziehen des ersten Öffnungsdraht (27) und des zweiten Öffnungsdrahts (29) betrieben werden, und in Schließrichtungen, sich einander annähernd, durch Ziehen des ersten Schließdrahts (28) und des zweiten Schließdrahts (30) betrieben werden;
der erste Klingenteil (22) vom ersten Basisabschnitt (19) in eine entgegengesetzte Richtung zur ersten Öffnungsrolle (20) und der ersten Schließrolle (21) vorspringt und der erste Klingenteil (22) aus einer Position zwischen der ersten Öffnungsrolle (20) und der ersten Schließrolle (21) und näher an der ersten Öffnungsrolle (20) vorspringt, sodass die erste Öffnungsrolle (20) näher an der ersten Klingenteilseite positioniert ist als der zweite Klingenteil (26) in Bezug auf die ersten Schließrolle (21) in eine Richtung, die orthogonal zur Öffnungs- und Schließrichtung ist;
der zweite Klingenteil (26) vom zweiten Basisabschnitt (23) in eine entgegengesetzte Richtung zur zweiten Öffnungsrolle (24) und der zweiten Schließrolle (25) vorspringt und der zweite Klingenteil (26) aus einer Position zwischen der zweiten Öffnungsrolle (24) und der zweiten Schließrolle (25) und näher an der zweiten Öffnungsrolle (24) vorspringt, sodass die zweite Öffnungsrolle (24) näher an der zweiten Klingenteilseite positioniert ist als der erste Klingenteil (22) in Bezug auf die zweite Schließrolle (25) in einer Richtung, die orthogonal zur Öffnungs- und Schließrichtung ist,
der erste Klingenteil (22) und der zweite Klingenteil (26) nebeneinander in einer Richtung angeordnet sind, die orthogonal zur Öffnungs- und Schließrichtung ist;
die zweite Öffnungsrolle (24) oder die zweite Schließrolle (25) zwischen der ersten Öffnungsrolle (20) und der ersten Schließrolle (21) positioniert ist; und
die erste Öffnungsrolle (20) oder die erste Schließrolle (21) zwischen der zweiten Öffnungsrolle (24) und der zweiten Schließrolle (25) positioniert ist, und **dadurch gekennzeichnet, dass**
die erste Öffnungsrolle (20), die zweite Schließrolle (25), die erste Schließrolle (21) und die zweite Öffnungsrolle (24) der Reihe nach in der axialen Richtung der Drehwelle (18) positioniert sind; und
in einer Richtung, die orthogonal zur Öffnungs- und Schließrichtung ist, die erste Öffnungsrolle (20) auf der ersten Klingenteilseite positioniert ist, und die zweite Öffnungsrolle (24) auf der zweiten Klingenteilseite positioniert ist.

2. Chirurgisches Instrument (1) gemäß Anspruch 1, wobei der erste Basisabschnitt (19), der erste Klingenteil (22), die erste Öffnungsrolle (20) und die erste Schließrolle (21) integral gebildet sind; und
der zweite Basisabschnitt (23), der zweite Klingenteil (26), die zweite Öffnungsrolle (24) und die zweite Schließrolle (25) integral gebildet sind.

3. Chirurgisches Instrument (1) gemäß Anspruch 1 oder Anspruch 2, wobei der Haltekörper (3) drehbar mit dem Basiskörper verbunden ist (2) mit einer Drehspindel (13) als Drehpunkt; und
die axiale Richtung der Drehwelle (18) und die axiale Richtung der Drehspindel (13) orthogonal zueinander sind.

## Revendications

1. Un instrument chirurgical (1) fixé à un bras robotique chirurgical, l'instrument chirurgical (1) comprenant :
un corps de base (2) détachable du bras robotique ;
un corps de support (3) relié au corps de base (2) ; et
un effecteur terminal (4) comprenant une première mâchoire (16) et une seconde mâchoire (17) chacune supportée de façon rotative par le corps de support (3) avec un arbre rotatif (18) comme pivot ; dans lequel
la première mâchoire (16) comprend une première poulie d'ouverture (20) à laquelle est relié un premier fil d'ouverture (27), une première poulie de fermeture (21) à laquelle est relié un premier fil de fermeture (28), et une première partie de lame (22) actionnée dans une direction d'ouverture et de fermeture, dans lequel la première mâchoire (16) est équipée d'une première section de base (19), et la première poulie d'ouverture (20) et la première poulie de fermeture (21) font saillie dans une même direction depuis les deux extrémités de la première section de base (19) ;
la seconde mâchoire (17) comprend une seconde poulie d'ouverture (24) à laquelle est relié un second fil d'ouverture (29), une seconde poulie de fermeture (25) à laquelle est relié un second fil de fermeture (30), et une seconde partie de lame (26) actionnée dans la direction d'ouverture et de fermeture, dans lequel la seconde mâchoire (17) est équipée d'une seconde section de base (23), et la seconde poulie d'ouverture (24) et la seconde poulie de fermeture (25) font saillie dans la même direction depuis les deux extrémités de la seconde section de base (23) ;
la première poulie d'ouverture (20) et la première poulie de fermeture (21) sont supportées par l'arbre rotatif (18) dans un état d'être séparée l'une de l'autre dans une direction axiale de l'arbre rotatif (18) ;
la seconde poulie d'ouverture (24) et la seconde poulie de fermeture (25) sont supportées par l'arbre rotatif (18) dans un état d'être séparée l'une de l'autre dans la direction axiale de l'arbre rotatif (18) ;
la première partie de lame (22) et la seconde partie de lame (26) sont actionnées dans des directions d'ouverture séparant l'une de l'autre en tirant respectivement le premier fil d'ouverture (27) et le second fil d'ouverture (29), et sont actionnées dans des directions de fermeture s'approchant l'une à l'autre en tirant respectivement le premier fil de fermeture (28) et le second fil de fermeture (30) ;
la première partie de lame (22) fait saillie de la première section de base (19) dans une direction opposée à la première poulie d'ouverture (20) et à la première poulie de fermeture (21) et la première partie de lame (22) fait saillie d'une position entre la première poulie d'ouverture (20) et la première poulie de fermeture (21), et plus près de la première poulie d'ouverture (20), de sorte que la première poulie d'ouverture (20) est positionnée plus près du côté de la première partie de lame que la seconde partie de lame (26) par rapport à la première poulie de fermeture (21) dans une direction orthogonale à la direction d'ouverture et de fermeture ;
la seconde partie de lame (26) fait saillie de la seconde section de base (23) dans une direction opposée à la seconde poulie d'ouverture (24) et à la seconde poulie de fermeture (25), et la seconde partie de lame (26) fait saillie d'une position entre la seconde poulie d'ouverture (24) et la seconde poulie de fermeture (25), et plus près de la seconde poulie d'ouverture (24), de sorte que la seconde poulie d'ouverture (24) est positionnée plus près du côté de la seconde partie de lame que la première partie de lame (22) par rapport à la seconde poulie de fermeture (25) dans une direction orthogonale à la direction d'ouverture et de fermeture,
la première partie de lame (22) et la seconde partie de lame (26) sont disposées côte à côte dans une direction orthogonale à la direction d'ouverture et de fermeture ;
la seconde poulie d'ouverture (24) ou la seconde poulie de fermeture (25) est positionnée entre la première poulie d'ouverture (20) et la première poulie de fermeture (21) ; et
la première poulie d'ouverture (20) ou la première poulie de fermeture (21) est positionnée entre la seconde poulie d'ouverture (24) et la seconde poulie de fermeture (25), et **caractérisé en ce que**
la première poulie d'ouverture (20), la seconde poulie de fermeture (25), la première poulie de fermeture (21) et la seconde poulie d'ouverture (24) sont positionnées dans l'ordre dans une direction axiale de l'arbre rotatif (18) ; et
dans une direction orthogonale à la direction d'ouverture et de fermeture, la première poulie d'ouverture (20) est positionnée du côté de la première partie de lame, et la seconde poulie d'ouverture (24) est positionnée du côté de la seconde partie de lame.

2. L'instrument chirurgical (1) selon la revendication 1, dans lequel la première section de base (19), la première partie de lame (22), la première poulie d'ouverture (20) et la première poulie de fermeture (21) sont formées de façon solidaire ; et
la seconde section de base (23), la seconde partie de lame (26), la seconde poulie d'ouverture (24) et la seconde poulie de fermeture (25) sont formées de façon solidaire.

3. L'instrument chirurgical (1) selon l'une des revendications 1 ou 2, dans lequel le corps de support (3) est relié de manière rotative au corps de base (2) avec un arbre pivot (13) servant de pivot ; et
la direction axiale de l'arbre rotatif (18) et la direction axiale de l'arbre pivot (13) sont orthogonales l'une à l'autre.
